(19) **Europäisches Patentamt**
**European Patent Office**
**Office européen des brevets**

(11) **EP 2 531 108 B1**

(12) **EUROPEAN PATENT SPECIFICATION**

(45) Date of publication and mention
of the grant of the patent:
**26.06.2013 Bulletin 2013/26**

(51) Int Cl.:
*A61B 6/12* *(2006.01)*       *A61B 19/00* *(2006.01)*

(21) Application number: **11706351.1**

(86) International application number:
**PCT/IB2011/050413**

(22) Date of filing: **31.01.2011**

(87) International publication number:
**WO 2011/095927 (11.08.2011 Gazette 2011/32)**

(54) **OBJECT LOCALIZATION APPARATUS**

GERÄT ZUR ORTUNG VON OBJEKTEN

APPAREIL DE LOCALISATION D'OBJET

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.02.2010 EP 10152604**

(43) Date of publication of application:
**12.12.2012 Bulletin 2012/50**

(73) Proprietors:
• **Koninklijke Philips Electronics N.V.
5621 BA Eindhoven (NL)**
Designated Contracting States:
**AL AT BE BG CH CY CZ DK EE ES FI FR GB GR
HR HU IE IS IT LI LT LU LV MC MK MT NL NO PL
PT RO RS SE SI SK SM TR**
• **Philips Intellectual Property & Standards GmbH
20099 Hamburg (DE)**
Designated Contracting States:
**DE**

(72) Inventors:
• **VAN STEVENDAAL, Udo
NL-5656 AE Eindhoven (NL)**
• **GRASS, Michael
NL-5656 AE Eindhoven (NL)**

(74) Representative: **Damen, Daniel Martijn
Philips
Intellectual Property & Standards
P.O. Box 220
5600 AE Eindhoven (NL)**

(56) References cited:
**DE-A1-102008 006 516     US-A1- 2003 130 576
US-A1- 2008 262 342     US-A1- 2009 171 196**

**Description**

FIELD OF THE INVENTION

The invention relates to an object localization apparatus, object localization method and object localization computer program for localizing an object having markers. The invention relates further to an object navigation apparatus, object navigation method and object navigation computer program, which include the object localization apparatus, object localization method and object localization computer program, respectively.

BACKGROUND OF THE INVENTION

In interventional radiology, fluoroscopy can be used for determining the position of a catheter within a person. An x-ray source and an x-ray detector are arranged such that radiation emitted by the x-ray source can traverse the person in which the catheter is located, and, after the radiation has traversed the person, the radiation can be detected by the x-ray detector for generating a projection image of the person. The x-ray radiation source and the x-ray detector are rotatable around the person for generating multiple projection images in different projection directions. The catheter is visible in the multiple projection images and the position of the catheter in the respective projection image is determined. These positions of the catheter in the different projection images are used for calculating the three-dimensional position of the catheter within the person.

This kind of determining the position of the catheter within the person requires generating several projection images in different projection directions. The generation of the several projection images interrupts the workflow of, for example, using the catheter for sensing or energy application purposes, and for each projection direction x-ray radiation has to be applied to the person, i.e. this kind of determining the position of the catheter within the person requires applying a significant radiation dose to the person.

SUMMARY OF THE INVENTION

It is an object of the present invention to provide an object localization apparatus, object localization method and object localization computer program, which allow localizing an object with a reduced number of projection images. It is a further object of the present invention to provide an object navigation apparatus, object navigation method and object navigation computer program, which include the object localization apparatus, object localization method and object localization computer program, respectively.

In a first object of the present invention an object localization apparatus for localizing an object having markers is presented, wherein the object localization apparatus comprises:

- a projection image providing unit for providing a projection image being generated by projecting the object with the markers in a projection plane,
- a projection image recognition unit for recognizing the projected markers and the projected object in the projection image,
- an object function providing unit for providing an object function along x-z positions, wherein the object function represents the object and the x-z positions are defined by pairs of an x position and a z position, wherein an x position is a position of a recognized projected marker along a fictive line on the recognized projected object in the projection plane and wherein a z position defmes a position in a direction being outside the projection plane,
- an object function adaptation unit for modifying the object function such that distances between the x-z positions along the object function are adapted to distances between the markers of the object by varying the z positions,
- a position determination unit for determining the position of the object based on the adapted object function and the positions of the recognized projected markers in the projection plane.

Since the position of the object is determined based on the adapted object function and the positions of the recognized projected markers in the projection plane and since the object function is adapted by varying the z positions such that distances between the x-z positions along the object function are adapted to distances between the markers of the object, this determination of the position of the object can be determined by using a reduced number of projection images. In particular, by using this kind of determining the position of the object, the object localization apparatus can be adapted to determine this position by using a single projection image only. Thus, if, for example, the object localization apparatus is used for determining the position of a catheter within a person, only a reduced number of projection images, in particular, only a single projection image, is needed for determining the position of the catheter. This reduces the number of interruptions of a workflow and decreases the radiation dose applied to the person.

The modification of the object function such that distances between the x-z positions along the object function are adapted to distances between the markers of the object by varying the z positions is preferentially performed such that after this modification the distances between the x-z positions are equal to the distances between the markers of the object.

The object is preferentially flexible. Moreover, the object is preferentially a catheter or another elongated object.

The position determination unit is preferentially adapted to determine the three-dimensional position of the object. Moreover, the position determination unit is preferentially adapted to determine the position, orientation and/or the run of the object based on the adapted object function and the positions of the recognized projected markers in the projection plane.

It is preferred that the object function adaptation unit is adapted to modify the object function such that the distances between the x-z positions along the object function are adapted to distances between the markers of the object along a fictive line, which follows the shape of the object and runs along, in particular, connects, the markers, by varying the z positions. For example, the fictive line may follow the shape of and may centrally be located within the object. In an embodiment, the object is a catheter having markers, wherein a wire is located within the catheter and runs along the markers, wherein this wire, which may be a steering wire, can define the fictive line of the catheter. The object function is preferentially a function representing the fictive line, in particular, representing a wire within a catheter.

The projection image providing unit can be a projection unit for projecting the object with the markers for generating the projection image in the projection plane. The projection unit is preferentially an x-ray projection unit comprising an x-ray source and an x-ray detector. The projection unit is, for example, an x-ray C-arm system or a computed tomography system. The x-ray source generates, for example, divergent light like a cone beam or parallel light. The projection unit can also be adapted to use another kind of radiation for generating the projection image. For example, nuclear radiation can be used for generating the projection image. Moreover, the projection image providing unit can also be a storing unit in which the projection image is stored already, or a projection image receiving unit for receiving the projection image, for example, via a wireless or wired data connection.

The markers are elements, which can be attached at fixed positions on the object and which are detectable in the projection image. For example, the markers are metal elements which can, for example, be clamped around the object, which is preferentially a catheter, or which can be attached to the object in another way, for example, by using screws or an adhesive.

A direction being outside the projection plane is a direction, which can be indicated by a direction vector, which cannot be placed completely within the projection plane. The direction being outside the projection plane can therefore be indicated by a direction vector having an angle not being zero to the projection plane. In particular, this direction is, for example, perpendicular to the projection plane or aligned with the ray direction of the rays being used for generating the respective projected marker. Thus, in an embodiment, if different markers have been projected in different ray directions, for example, because divergent radiation has been used for generating the projection image, the direction outside the projection plane can be different for different markers.

The fictive line on the projected object is preferentially a fictive line following the shape of the projected object in the projection plane and directly connecting neighbored projected markers. For example, if the projected object is a projected curved catheter, the fictive line follows the curvature of the projected catheter. In particular, if the catheter comprises a wire running along the markers, the fictive line on the projected catheter can represent the projected wire of the catheter. Similarly, as already mentioned above, the fictive line of the object is preferentially a fictive line following the shape of the object and running along the markers. For example, if the object is a curved catheter, the fictive line follows the curvature of the catheter, in particular, the fictive line can represent a wire located within the catheter and running along the different markers.

It is preferred that the object function providing unit is adapted to provide a spline line function connecting neighbored x-z positions as the object function. The spline line function is preferentially a cubic spline line function, in particular, a natural cubic spline line function.

It is further preferred that the object function comprises at least one object function element forming the object function, wherein the object comprises at least two markers, wherein a object function element connects two neighbored x-z positions, wherein the object function adaptation unit is configured to adapt the object function such that at least one of the following conditions is fulfilled:

- two object function elements have the same value at an x-z position, which has two neighbored x-z positions,
- the first derivative of two object function elements is equal at an x-z position, which has two neighbored x-z positions,
- the second derivative of two object element function is equal at an x-z position, which has two neighbored x-z positions,
- the second derivative of an object function element is zero at an x-z position, which has only one neighbored x-z position. This allows adapting the object function with small computational efforts in a simple way.

Preferentially, the object function is adapted such that at each x-z position the respective conditions, i.e. the conditions for two neighbored x-z positions or the condition for only a single neighbored x-z position, are fulfilled. The object function element is preferentially a spline line element, in particular, a cubic spline line element, further preferred a natural cubic spline line element. Neighbored preferentially means directly neighbored.

It is preferred that the object localization apparatus further comprises a marker position providing unit for providing a marker position of at least one of the markers of the object, wherein the object function adaptation unit is configured to determine an x-z position, which corresponds to the provided marker position depending on the provided marker position and the position of the corresponding recognized projected marker in the projection plane, and to adapt the object function such that the determined x-z position lies on the adapted object function. The position of the recognized projected marker along the fictive line in the projection plane defines the x position of this marker. The z position of the marker in a direction being outside the projection plane, in particular, in a direction perpendicular to the projection plane, can be used as reference, i.e. for example, can be set to zero. However, the z position can also be set to another value, for example, to a distance between the respective marker and the projection plane in the z direction. It is further preferred that the object has a maximal possible curvature, wherein the object function adaptation unit is configured to adapt the object function such that its curvature is not larger than the maximal possible curvature of the object. It is also preferred that the object localization apparatus comprises an envelope providing unit for providing an envelope defming possible positions of the object function, wherein the object function adaptation unit is configured to adapt the object function such that it is located within the envelope. These further conditions can be used to improve the accuracy of the determined position and/or for determining the position with reduced computational efforts, because certain positions are excluded by these conditions, for example, because they are not within the envelope or they do not correspond to the maximal possible curvature or not to the marker position of the at least one of the markers.

It is further preferred that the position determination unit is adapted to determine the position of the object by

- determining two dimensions of the positions of the markers as the two-dimensional positions of the recognized projected markers in the projection plane,
- determining a third dimension of the positions of the markers as the z positions of x-z positions, which correspond to the markers, along the adapted object function,
- arranging the adapted object function such that the x-z positions along the adapted object function coincide with the determined three-dimensional positions of the markers. This allows determining the position, orientation and run of the object fast and with low computational effort.

In a further aspect of the present invention an object navigation apparatus is presented, wherein the object navigation apparatus comprises:

- an object having markers,
- an object localization apparatus as defined in claim 1 for localizing the object, wherein the position of the object is determined,
- an object navigating unit for navigating the object depending on the determined position of the object.

The object is preferentially a catheter and, thus, the object navigation apparatus is preferentially an apparatus for navigating a catheter within a person, for example, within a heart of a person. The catheter can comprise further elements like sensing elements for sensing the interior of a person like electrical or optical sensing elements and/or energy application elements like ablation elements for ablating the interior of the person, for example, by using electrical or optical energy or by using coldness or heat.

In a further aspect of the present invention an object localization method for localizing an object having markers is presented, wherein the object localization method comprises:

- providing a projection image being generated by projecting the object with the markers in a projection plane,
- recognizing the projected markers and the projected object in the projection image,
- providing an object function along x-z positions wherein the object function represents the object and the x-z positions are defmed by pairs of an x position and a z position, wherein an x position is a position of a recognized projected marker along a fictive line on the recognized projected object in the projection plane and wherein a z position defines a position in a direction being outside the projection plane,

- modifying the object function such that distances between the x-z positions along the object function are adapted to distances between the markers of the object by varying the z positions,
- determining the position of the object based on the adapted object function and the positions of the recognized projected markers in the projection plane.

In a further aspect of the present invention an object navigation method is presented, wherein the object navigation method comprises:

- providing an object having markers,
- localizing the object by the object localization method defined in claim 8, wherein the position of the object is determined,
- navigating the object depending on the determined position of the object.

In a further aspect of the present invention an object localization computer program for localizing an object having markers is presented, wherein the object localizing computer program comprises program code means for causing a computer to carry out the steps of the object localizing method as defined in claim 10, when the computer program is run on a computer controlling an object localizing apparatus as defined in claim 1.

In a further aspect of the present invention an object navigation computer program is presented, wherein the object navigation computer program comprises program code means for causing a computer to carry out the steps of the object navigation method as defined in claim 11, when the computer program is run on a computer controlling an object navigation apparatus as defined in claim 9.

It shall be understood that the object localization apparatus of claim 1, the object navigation apparatus of claim 9, the object localization method of claim 10, the object navigation method of claim 11, the object localization computer program of claim 12 and the object navigation computer program of claim 13 have similar and/or identical preferred embodiments, in particular, as defined in the dependent claims.

It shall be understood that a preferred embodiment of the invention can also be any combination of the dependent claims with the respective independent claim.

These and other aspects of the invention will be apparent from and elucidated with reference to the embodiments described hereinafter.

## BRIEF DESCRIPTION OF THE DRAWINGS

In the following drawings:

Fig. 1 shows schematically and exemplarily an embodiment of an object localization apparatus,
Fig. 2 shows schematically and exemplarily a distal end of a catheter comprising markers,
Fig. 3 exemplarily illustrates the generation of a projection image,
Fig. 4 shows schematically and exemplarily an object function representing an object,
Fig. 5 shows schematically and exemplarily an embodiment of an object navigation apparatus,
Fig. 6 shows exemplarily a flowchart illustrating an embodiment of an object localization method, and
Fig. 7 shows exemplarily a flowchart illustrating an embodiment of an object navigation method.

## DETAILED DESCRIPTION OF EMBODIMENTS

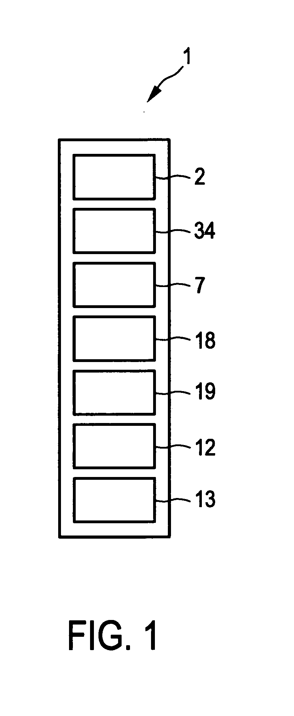

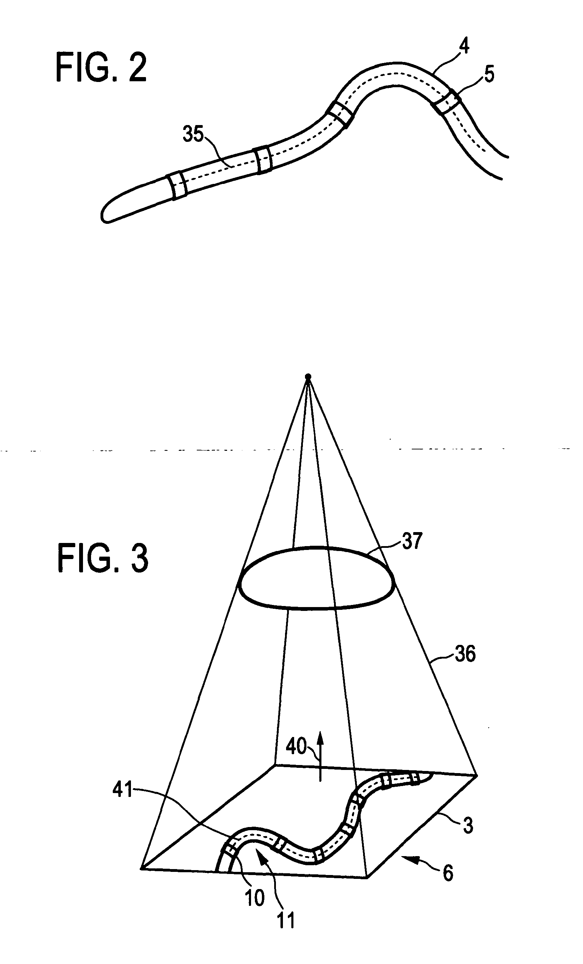

Fig. 1 shows schematically and exemplarily an object localization apparatus 1 for localizing an object having markers. In this embodiment the object is a flexible catheter 4 having markers 5. A distal end of this catheter 4 is schematically and exemplarily shown in Fig. 2. The markers are preferentially equidistantly arranged along a fictive line 35 on the catheter 4 connecting the markers 5. In another embodiment, the markers can also be arranged on the catheter non-equidistantly. The markers 5 are attached at fixed positions on the catheter 4 and are detectable in a projection image. In particular, the markers 5 are metal elements which, for example, can be clamped around the catheter 4 or which, for example, can be attached to the catheter in another way, for example, by using screws or an adhesive. The fictive line 35 on the catheter 4 follows the curvature of the catheter 4 and can be defined by a wire located within the catheter 4 and running along the markers 5. For example, the wire can be a steering wire.

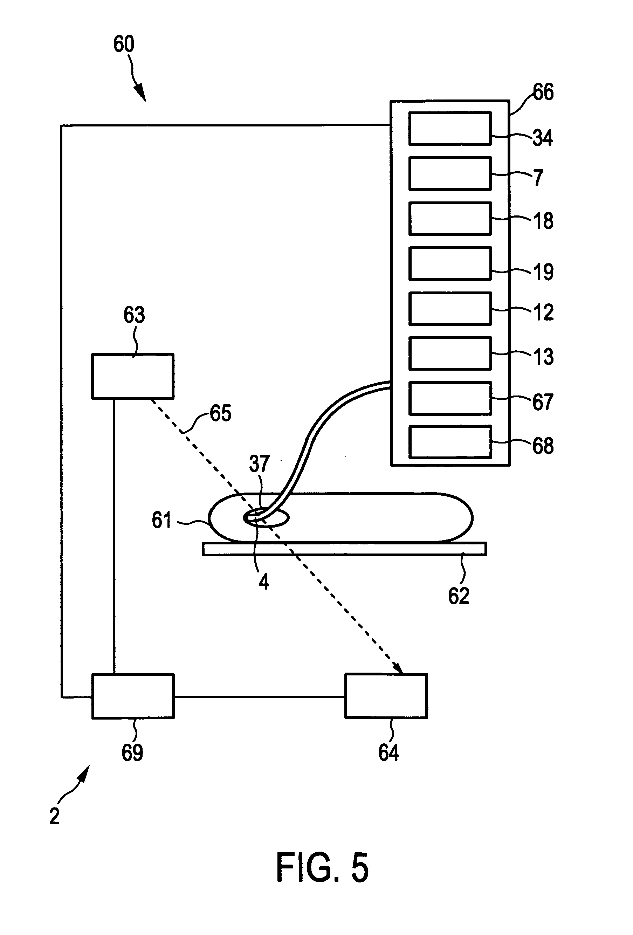

The object localization apparatus 1 comprises a projection image providing unit 2 for providing a projection image being generated by projecting the catheter 4 with the markers 5 in a projection plane. The generation of the projection image is schematically and exemplarily illustrated in Fig. 3. A radiation source generates, for example, a cone beam 36 for traversing an organ 37 of a person, or another subject in which the catheter 4 (not shown in Fig. 3) is located. The cone beam, which has traversed the organ 37, is detected by a radiation detector in a projection plane 6 for generating a projection image 3 showing the projected markers 10 and the projected catheter 11. The projection image providing unit 2 can be a storing unit in which the projection image 6 is stored already, or a projection image receiving unit for receiving the projection image 3, for example, via a wireless or wired data connection, from, for instance, a projection unit like an x-ray C-arm system or a computed tomography system. However, the projection image providing unit 2 can also be the projection unit itself, i.e. the projection image providing unit 2 can also be, for example, an x-ray C-arm system or a computed tomography system. Such a projection unit is exemplarily described further below with reference to Fig. 5.

The object localization apparatus 1 further comprises a projection image recognition unit 35 for recognizing the projected markers 10 and the projected object 11 in the projection image 3 and, thus, in the projection plane 6. The projection image recognition unit 35 can be adapted to recognize a part of the projected object 11 only. For example, if the projected object is a projected catheter 11 comprising a wire running along the projected markers, the projection image recognition unit 35 can be adapted to recognize the projected wire in the projection image.

The projected markers 10 and the projected object 11 are preferentially recognized by segmenting the same in the projection image, for example, by using a thresholding method. Methods for recognizing projected markers and projected objects in a projection image are well known and are disclosed, for example, in "3D Reconstruction of Coronary Stents in Vivo Based on Motion Compensated X-Ray Angiograms" by Babak Movassaghi, MICCAI 2006, LNCS 4191, pages 177-184 (2006); and "Scale-Space Signatures for the Detection of Clustered Microcalcifications in Digital Mammograms" by T. Netsch and H. O. Peitgen, IEEE Trans. Med. Imag., vol. 18, no. 9, pages 774 to 786 (1999).

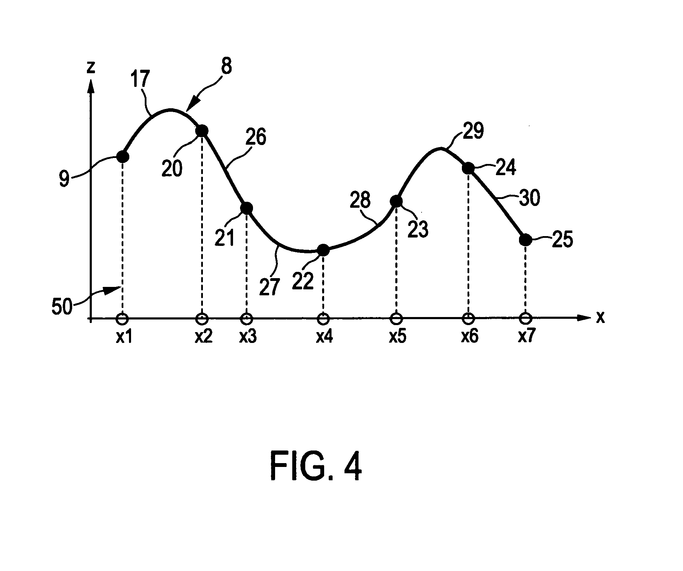

The object localization apparatus 1 further comprises an object function providing unit 7 for providing an object function representing the catheter 4. In this embodiment, the object function 8, which is schematically exemplarily shown in Fig. 4, is a spline line function connecting neighbored x-z positions 9, 20, 21, 22, 23, 24. The spline function 8 comprises several spline line elements 17, 26, 27, 28, 29, 30 forming the object function 8. The spline line elements are preferentially cubic spline line elements and further preferred natural cubic spline line elements. Each spline line element connects two neighbored x-z positions. The object function 8 depends therefore on the x-z positions.

An x-z position is defmed by a pair of an x position and a z position, wherein an x position is a position of a recognized projected marker 10 along a fictive line 41 on the recognized projected object 11 in the projection plane 6. In Fig. 4, x positions are exemplarily indicated by xl...x7. A z position defines a position in a direction 40 being outside the projection plane 6. The fictive line 41 of the projected catheter 11 follows the shape of the projected catheter 11 in the projection plane 6 and directly connects neighbored projected markers. The projected catheter has a snake-like shape and the fictive line 41 extends preferentially along the center of the snake-like shape of the projected catheter 11. However, the fictive line 41 can also follow another path on the snake-like shape of the projected catheter 11. If the catheter comprises a wire running along the markers and if this wire is recognizable in the projection image, the fictive line 41 preferentially follows the projected wire in the projection plane and the object function represents preferentially this wire within the catheter.

The direction 40 being outside the projection plane 6 is, in this embodiment, perpendicular to the projection plane 6 and points into the direction of a radiation source generating the cone beam 36. However, in another embodiment, the direction being outside the projection plane can also be indicated by a direction vector having an angle not being zero to the projection plane 6 and not being perpendicular to the projection plane 6. For example, the direction vector can be aligned with the ray direction of the ray, which has been used for generating the respective-projected marker 10. Since different markers are projected by different rays, which are differently oriented, in this case the direction outside the projection plane 6 can be different for different projected markers 10.

The object localization apparatus 1 further comprises an object function adaptation unit 12 for modifying the object function 8. The object function adaptation unit 12 is preferentially configured to adapt the object function 8 such that following conditions are fulfilled: the respective two spline line elements at the x-z positions 20, 21, 22, 23, 24, which each have two neighbored x-z positions, have the same value, the same first derivative, and the same second derivative, and the second derivative of a respective spline line element is zero at the x-z positions 9, 25, which only have one neighbored x-z position.

The object localization apparatus 1 further comprises a marker position providing unit 18 for providing a marker position of at least one of the markers 5 on the catheter 4. The marker position providing unit 18 is, for example, a storing unit in which the marker position, which has already been determined, is stored, or a receiving unit for receiving an already determined marker position, for example, via a wired or wireless data communication. Without loss of generality, the position of a marker could be obtained by a registered three-dimensional left atrium surface model with a marked position of a transseptal puncture supported by a rotational atriography run, if the catheter 4 is located within a heart of a person. The catheter can be arranged such that a marker 5 is located at a location at which the catheter has entered the left atrium, wherein this location is known from the registered three-dimensional left atrium surface model. Generally, if a catheter is introduced into a subject like an organ of a person and if a model of the subject with a known position has been generated and if the position of at least one marker with respect to the model is known, this known marker position can be provided by the marker position providing unit.

Since the position of the at least one marker 5 is known and since also the position of the projected marker 10 in the projection plane 6 is known, the x-z position, which corresponds to this marker, can be determined, wherein the object function is adapted such that this determined x-z position, which corresponds to the provided marker position of the at least one marker, lies on the adapted object function.

The object function adaptation unit 12 is configured to modify the object function 8 such that distances between the x-z positions 9, 20...25 along the object function 8 connecting the x-z positions 9, 20...25 are adapted to distances between the markers 5 on the catheter 4 along the fictive line 35 on the catheter 4 connecting the markers 5 by varying the z positions. Thus, the x-z positions 9, 20...25 are moved along the dashed vertical lines 50 shown in Fig. 4 such that the distances between the x-z positions 9, 20....25 are equal to the distances between the markers 5 on the catheter 4 and the above mentioned conditions are fulfilled.

The determination of the object function will be described in the following exemplarily for a case in which a catheter 4 comprises four markers 5, which are recognized in the projection image.

In this example, the object function is comprised of three spline line elements connecting four x-z positions, which correspond to the four markers. A spline line element may be defined by following equation:

$$z_j(x) = a_j + b_j(x - x_j) + c_j(x - x_j)^2 + d_j(x - x_j)^3 \quad , \tag{1}$$

wherein the index $j$ indicates the respective spline line element, $x_j$ indicates the respective x position of the respective projected marker, and $a_j$, $b_j$, $c_j$, $d_j$ are the parameters defining the $j$-th spline line element. Each spline element starts at a first x position and ends at a second x position, wherein the first x position is smaller than the second x position. For a spline line element indicated by the index $j$, $x_j$ preferentially indicates the corresponding first x position. For determining the object function, three times four, i.e. twelve, parameters have to be determined in this example with four markers and three spline line elements. Since, according to the above mentioned conditions, at the two inner x-z positions the respective two spline line elements have to have the same value, the same first derivative and the same second derivative, these conditions yield six equations. Moreover, since at the two outer x-z positions the second derivative should be zero, two more equations are obtained. Since the three distances along the object function between the four x-z positions should be equal to the distances between the markers 5 along the fictive line 35 on the catheter 4, three more equations are obtained, and a twelfth equation is given by using the condition that the x-z position defined by the provided marker position should lie on the adapted object function. Thus, in this embodiment the above mentioned conditions provide twelve equations which can be used for determining the twelve parameters of the three spline line elements defining the object function.

For determining the object function 8 further boundary conditions can be added in order to confine the solution. For example, the maximum curvature of the catheter 4 can be a given physical condition, wherein the object function adaptation unit 12 can be configured to adapt the object function 8 such that its curvature is not larger than the maximal possible curvature of the catheter 4. Moreover, the object localization apparatus 1 can further comprise an envelope providing unit 19 for providing an envelope defining possible positions of the object function, wherein the object function adaptation unit 12 may be configured to adapt the object function 8 such that it is located within the envelope. For example, if the catheter 4 is located within the heart, the shape of the atrium, which might have been determined in advance by a rotational atriography run, could define an envelope. In addition or alternatively, also the projected size of the catheter 4 and of the markers 5 on the projection image could serve as a boundary condition for the equation system, i.e. based on the known projection geometry and the dimensions of the projected catheter and projected markers a three-dimensional region can be defined, in which the catheter can be located only. This three-dimensional region limits the space of possible x-z positions.

The object localization apparatus 1 further comprises a position determination unit 13 for determining the three-dimensional position of the catheter 4 based on the adapted object function 8 and the positions of the recognized projected markers 10 in the projection plane 6. The position determination unit 13 is preferentially adapted to not only determine the position of the catheter 4, but also the orientation and the run of the catheter 4 based on the adapted object function 8 and the positions of the recognized projected markers 10 in the projection plane 6. For determining the position, orientation and run of the catheter 4 in three dimensions, the three-dimensional positions of the markers 5 are determined. Two dimensions of a marker 5 are determined as being the position of the corresponding projected marker 10 in the projection plane 6, and the third dimension is the z position of the adapted object function 8 at the x position which corresponds to the respective marker. These determined three-dimensional positions of the markers 5 correspond to the respective x-z positions of the object function 8 such that the adapted object function 8 can be arranged in the three-dimensional space such that the determined three-dimensional marker positions lie on the adapted object function 8, wherein the arranged adapted object function 8 is the determined position, orientation and run of the catheter 4.

Fig. 5 shows schematically and exemplarily an object navigation apparatus 60 comprising an object being the catheter 4 with the markers 5. The catheter 4 is introduced into an organ 37 like the heart of a person 61 who is located on a subject table 62. The object navigation apparatus 60 comprises an object localization apparatus as described above for localizing the catheter 4, in particular, for determining the position and optionally the orientation and run of the catheter 4. That means the object navigation apparatus 60 comprises the projection image recognition unit 34, the object function providing unit 7, optionally the marker position providing unit 18, optionally the envelope providing unit 19, the object function adaptation unit 12 and the position determination unit 13. The object localization apparatus 60 also comprises the projection image providing unit 2, which is, in this embodiment, an x-ray projection unit comprising an x-ray source 63 and an x-ray detector 64. The x-ray projection unit 2 is preferentially an x-ray C-arm system or a computed tomography system. The x-ray source 63 generates light 65 being, for example, divergent light like a cone beam or parallel light, wherein the light 65 is detected by the x-ray detector 64 after having traversed the person 61. The projection image providing unit can also be another kind of projection unit which uses, for example, nuclear radiation for generating the projection image. The x-ray source 63 and the x-ray detector 64 are preferentially controlled by a projection unit control unit 69.

The projection image recognition unit 34, object function providing unit 7, marker position providing unit 18, envelope providing unit 19, object function adaptation unit 12 and position determination unit 13 are part of a catheter control unit 66. The catheter control unit 66 further comprises a catheter navigation unit 67 for navigating the catheter 4 within the organ 37 of the person 61 depending on the determined position and preferentially also depending on the determined orientation and run of the catheter 4.

The catheter navigation unit 67 is preferentially adapted to navigate the catheter 4, in particular, the distal end of the catheter 4, to a desired location within the organ 37 of the person 61 depending on the determined position and optionally orientation and run of the catheter 4. The navigation unit 61 can be adapted to allow a user to navigate the catheter 4 completely by hand or semi- or fully-automatically depending on the determined position and preferentially orientation and run of the catheter 4. The catheter 4 preferentially comprises build-in guiding means which can be controlled by the catheter navigation unit 61. The catheter 4 can, for example, be steered and navigated by the use of steering wires in order to guide, for example, the distal end of the catheter 4 to the desired location within the organ 37. However, the catheter navigation unit and the catheter can also be adapted to be navigated by using magnetic forces, i.e. the catheter navigation unit and the catheter can be adapted to be magnetically navigated.

The catheter 4 preferentially comprises further elements for sensing the organ 37 and/or for applying energy to the organ 37. The catheter control unit 31 can comprise a corresponding sensing and/or energy application unit 68 for controlling sensing of and/or energy application to the organ 37. The sensing and/or energy application elements within the catheter 4 and the corresponding sensing and/or energy application unit 68 can be adapted to, for example, optically or electrically sense the interior of the organ 37. Also ultrasound could be used for sensing the interior of the organ 37. They can also be used for applying, for example, electrical or optical energy to the interior of the organ 37, for instance, for ablation purposes.

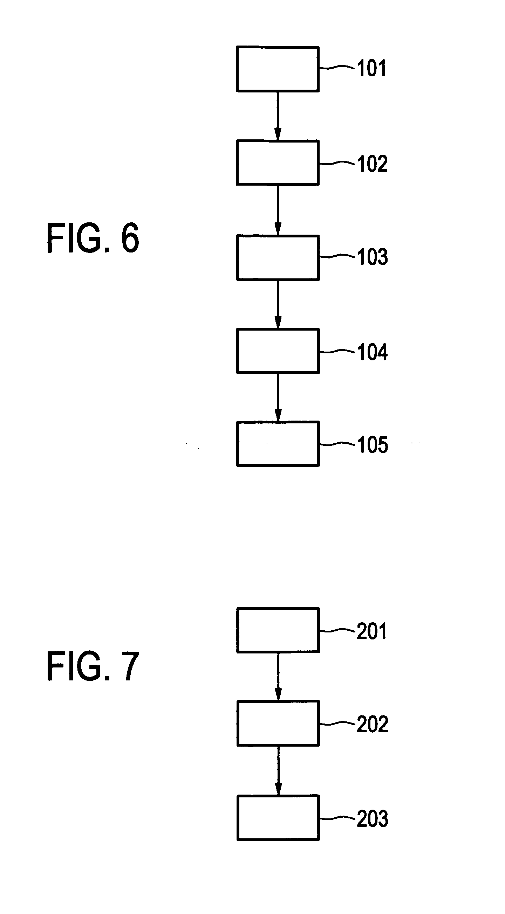

In the following an object localization method will exemplarily be described with reference to a flowchart shown in Fig. 6.

In step 101, a projection image 3 being generated by projecting the catheter 4 with the markers 5 in the projection plane 6 is provided by the projection image providing unit 2. In step 102, the projected markers 10 and the projected object 11 are recognized in the projection image 3 by the projection image generation unit 34. In step 103, an object function 8 representing the catheter 4 is provided, wherein the object function 8 depends on x-z positions 9, 20...25 defined by pairs of an x position and a z position, wherein an x position is the position of a recognized projected marker 10 along a fictive line 41 on the recognized projected catheter 11 in the projection plane 6 and wherein a z position defines a position in a direction 40 being outside the projection plane 6. In step 104, the object function 8 is modified such that distances between the x-z positions 9, 20...25 along the object function 8 connecting the x-z positions 9, 20... 25 are adapted to distances between the markers 5 on the catheter 4 along a fictive line 35 on the catheter 4 connecting the markers 5 by varying the z positions, and in step 105 the position and preferentially also the orientation and run of the catheter are determined based on the adapted object function 8 and the positions of the recognized projected markers 10 in the projection plane 6.

In the following an embodiment of an object navigation method will exemplarily be described with reference to a flowchart shown in Fig. 7.

In step 201, a catheter 4 having markers 5 is provided, for example, within the organ 37 of the person 61. In step 202, the catheter 4 is localized by the object localization method described above with reference to Fig. 6, wherein the position and preferentially also the orientation and run of the catheter 4 are determined, and in step 203 the catheter 4 is navigated depending on the determined position and preferentially orientation and run of the catheter 4. Steps 202 and 203 can be performed in a loop or simultaneously, wherein the actually determined position and preferentially orientation and run of the catheter 4 are used for navigating the catheter 4 preferentially to a desired location.

The object localization apparatus can comprise further elements for determining the position of the catheter like electromagnetic tracking devices, which can be combined with the determination of the position using the above described object function, for increasing the accuracy of determining the position, in particular, the three-dimensional position, of the object.

The object navigation apparatus can be adapted for minimal invasive interventions performed under x-ray guidance which generally require strong three-dimensional/four-dimensional localization capabilities. The object localization apparatus preferentially uses only a single projection image in which the markers of the object are recognizable for three-dimensionally localizing the object. The object localization apparatus is preferentially adapted to perform the localization in real-time. In an embodiment, a single projection of a three-dimensional catheter generates a two-dimensional reproduction, i.e. a projection image, on a flat panel detector. Using the reproduction, the arc lines from projected marker to projected marker along the projected catheter is measured, which yields the x positions of the projected markers. For these projected markers, the z positions can be regarded as being zero. The catheter can now be considered as the representation of several natural cubic splines connected at the marker points in a two-dimensional plane and forming the object function in this two-dimensional plane. The two-dimensional plane is defined by the x and z positions. Cubic splines are continuous and differential. The first and second derivative of two splines must be equal in each connection point. This leads to numerous equations. If, for example, four marker points are considered, twelve coefficients of three cubic splines are preferentially calculated as described above.

The described object localization apparatus and object navigation apparatus preferentially allow catheter localization and low-dose catheter tracking. Possible modifications are, for example, a coupling of the equation system across several catheter position measurements in order to achieve a more stable solution or to generate a three-dimensional model of the bounding surface from the x-ray projection itself.

The fictive line of the object is preferentially a line running along, in particular, connecting, adjacent markers of the object, wherein the line follows the shape of the object. This fictive line preferentially runs along the centers of the adjacent markers. The fictive line of the object can be located on a surface of the object, in particular, on an outer surface, or the fictive line of the object can be located within, in particular, centrally within, the object. The fictive line on the projected object is preferentially located within the projected object, in particular, centrally within the projected object. If the object is a catheter comprising a wire running along the markers, preferentially the object function represents this wire, the fictive line of the object represents also this wire and the fictive line of the projected catheter represents the projected wire.

**[0057]** - Although in the above described embodiments a catheter having a certain number of markers has been described, in other embodiments the catheter can also comprise another number of markers. Also the shape of the markers can be different, and the object localization apparatus can be adapted to localize another kind of object not being a catheter. For example, the object can be a bendable interventional needle or a minimal-invasive imaging device like an intra-cardiac echo probe.

Although in the above described embodiments the catheter is preferentially localized within an organ of a person, the object localization apparatus can also be adapted to localize the catheter or another object within another element, for example, within another part of the person like the vessels, or within a technical object like a pipeline or another hollow technical element.

Although in the above described embodiments, the object function is a spline line function comprising several spline line elements, in other embodiments the object function can also be another function representing the object. For example, a polynomial can be used for representing the object.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Provisions, adaptations and determinations like the provision of an object function, the adaptation of the object function and the determination of the position of the object performed by one or several units or devices can be performed by any other number of units or devices. For example, steps 102 to 105 can be performed by a single unit or by any other number of different units. The provisions, adaptations and determination and/or the control of the object localization apparatus depending on the object localization method and/or the control of the object navigation apparatus in accordance with the object navigation method can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to an object localization apparatus for localizing an object having markers based on a projection image. An object function is provided along x-z positions, wherein the x-z positions are defmed by pairs of an x position being a position of a recognized projected marker along a fictive line on the recognized projected object in a projection plane and a z position defming a position in a direction being outside the projection plane. The position is determined based on the object function which is modified by varying the z positions such that distances between the x-z positions along the object function are adapted to distances between the markers of the object. This allows the object localization apparatus to determine the position with a reduced number of projection images, in particular, with only a single projection image.

**Claims**

1. An object localization apparatus for localizing an object having markers, the object localization apparatus (1) comprising:

   - a projection image providing unit (2) for providing a projection image (3) being generated by projecting the object (4) with the markers (5) in a projection plane (6),
   - a projection image recognition unit (34) for recognizing the projected markers (10) and the projected object (11) in the projection image (3),
   - an object function providing unit (7) for providing an object function (8) along x-z positions (9, 20...25), wherein the object function (8) represents the object (4) and the x-z positions (9, 20...25) are defined by pairs of an x position and a z position, wherein an x position is a position of a recognized projected marker (10) along a fictive line (41) on the recognized projected object (11) in the projection plane (6) and wherein a z position defines a position in a direction (40) being outside the projection plane (6),
   - an object function adaptation unit (12) for modifying the object function (8) such that distances between the x-z positions (9, 20...25) along the object function (8) are adapted to distances between the markers (5) of the object (4) by varying the z positions,
   - a position determination unit (13) for determining the position of the object (4) based on the adapted object function and the positions of the recognized projected markers in the projection plane.

2. The object localization apparatus as defined in claim 1, wherein the object function adaptation unit (12) is adapted to modify the object function (8) such that the distances between the x-z positions (9, 20...25) along the object function (8) are adapted to distances between the markers (5) of the object (4) along a fictive line (35), which follows the shape of the object (4) and runs along the markers (5), by varying the z positions.

3. The object localization apparatus as defined in claim 1, wherein the object function providing unit (7) is preferentially adapted to provide a spline line function connecting neighbored x-z positions (9, 20...25) as the object function (8).

4. The object localization apparatus as defined in claim 1, wherein the object function (8) comprises at least one object function element (17, 26 ... 30) forming the object function (8), wherein the object (4) comprises at least two markers (5), wherein a object function element (17, 26 ... 30) connects two neighbored x-z positions, wherein the object function adaptation unit (12) is configured to adapt the object function (8) such that at least one of the following conditions is fulfilled:

   - two object function elements have the same value at an x-z position, which has two neighbored x-z positions,
   - the first derivative of two object function elements is equal at an x-z position, which has two neighbored x-z positions,
   - the second derivative of two object element function is equal at an x-z position, which has two neighbored x-z positions,
   - the second derivative of an object function element is zero at an x-z position, which has only one neighbored x-z position.

5. The object localization apparatus as defined in claim 1, wherein the object localization apparatus (1) further comprises a marker position providing unit (18) for providing a marker position of at least one of the markers (5) on the object (4), wherein the object function adaptation unit (12) is configured to determine an x-z position, which corresponds to the provided marker position depending on the provided marker position and the position of the corresponding recognized projected marker in the projection plane, and to adapt the object function such that the determined x-z position (9) lies on the adapted object function.

6. The object localization apparatus as defined in claim 1, wherein the object (4) has a maximal possible curvature and wherein the object function adaptation unit (12) is configured to adapt the object function (8) such that its curvature is not larger than the maximal possible curvature of the object (4).

7. The object localization apparatus as defined in claim 1, wherein the object localization apparatus (1) further comprises an envelope providing unit (19) for providing an envelope defining possible positions of the object function, wherein the object function adaptation unit (12) is configured to adapt the object function such that it is located within the envelope.

8. The object localization apparatus as defined in claim 1, wherein the position determination unit (13) is adapted to determine the position of the object (4) by

   - determining two dimensions of the positions of the markers (5) as the two-dimensional positions of the recognized projected markers (10) in the projection plane (6),
   - determining a third dimension of the positions of the markers (5) as the z positions of x-z positions, which correspond to the markers (5), along the adapted object function (8),
   - arranging the adapted object function (8) such that the x-z positions along the adapted object function (8) coincide with the determined three-dimensional positions of the markers (5).

9. An object navigation apparatus comprising:

   - an object (4) having markers (5),
   - an object localization apparatus (1) as defined in claim 1 for localizing the object, wherein the position of the object is determined,
   - an object navigating unit (67) for navigating the object (4) depending on the determined position of the object.

10. An object localization method for localizing an object having markers, the object localization method comprising:

   - providing a projection image (3) being generated by projecting the object (4) with the markers (5) in a projection plane (6),
   - recognizing the projected markers (10) and the projected object (11) in the projection image (3),
   - providing an object function (8) along x-z positions (9, 20...25), wherein the object function (8) represents the object (4) and the x-z positions (9, 20...25) are defined by pairs of an x position and a z position, wherein an x position is a position of a recognized projected marker (10) along a fictive line on the recognized projected object (11) in the projection plane (6) and wherein a z position defines a position in a direction (40) being outside the projection plane (6),
   - modifying the object function (8) such that distances between the x-z positions (9, 20...25) along the object function (8) are adapted to distances between the markers (5) of the object (4) by varying the z positions,
   - determining the position of the object (4) based on the adapted object function and the positions of the recognized projected markers in the projection plane.

11. An object navigation method comprising:

   - providing an object (4) having markers (5),
   - localizing the object (4) by the object localization method defined in claim 10, wherein the position of the object (4) is determined,
   - navigating the object (4) depending on the determined position of the object (4).

12. An object localization computer program for localizing an object having markers, the object localizing computer program comprising program code means for causing a computer to carry out the steps of the object localizing method as defined in claim 10, when the computer program is run on a computer controlling an object localizing apparatus as defined in claim 1.

13. An object navigation computer program, the object navigation computer program comprising program code means for causing a computer to carry out the steps of the object navigation method as defined in claim 11, when the computer program is run on a computer controlling an object navigation apparatus as defined in claim 9.

**Patentansprüche**

1. Objektortungsgerät zur Ortung eines Objekts mit Markierungen, wobei das Objektortungsgerät (1) Folgendes umfasst:

   - eine Projektionsbildbereitstellungseinheit (2) zum Bereitstellen eines Projektionsbildes (3), das erzeugt wird, indem das Objekt (4) mit den Markierungen (5) in eine Projektionsebene (6) projiziert wird,
- eine Projektionsbilderkennungseinheit (34) zum Erkennen der projizierten Markierungen (10) und des projizierten Objekts (11) in dem Projektionsbild (3),
- eine Objektfunktionsbereitstellungseinheit (7) zum Bereitstellen einer Objektfunktion (8) entlang x-z-Positionen (9, 20...25), wobei die Objektfunktion (8) das Objekt (4) darstellt und die x-z-Positionen (9, 20...25) durch Paare aus einer x-Position und einer z-Position definiert werden, wobei eine x-Position eine Position einer erkannten projizierten Markierung (10) entlang einer fiktiven Linie (41) auf dem erkannten projizierten Objekt (11) in der Projektionsebene (6) ist und wobei eine z-Position eine Position in einer Richtung (40) definiert, die außerhalb der Projektionsebene (6) liegt,
- eine Objektfunktionsanpassungseinheit (12), um die Objektfunktion (8) derartig zu modifizieren, dass Abstände zwischen den x-z-Positionen (9, 20...25) entlang der Objektfunktion (8) an Abstände zwischen den Markierungen (5) des Objekts (4) angepasst werden, indem die z-Positionen variiert werden,
- eine Positionsbestimmungseinheit (13) zum Bestimmen der Position des Objekts (4) basierend auf der angepassten Objektfunktion und den Positionen der erkannten projizierten Markierungen in der Projektionsebene.

2. Objektortungsgerät nach Anspruch 1, wobei die Objektfunktionsanpassungseinheit (12) dafür ausgelegt ist, die Objektfunktion (8) derartig zu modifizieren, dass die Abstände zwischen den x-z-Positionen (9, 20...25) entlang der Objektfunktion (8) an Abstände zwischen den Markierungen (5) des Objekts (4) entlang einer fiktiven Linie (35) angepasst werden, die der Form des Objekts (4) folgt und entlang den Markierungen (5) verläuft, indem die z-Positionen variiert werden.

3. Objektortungsgerät nach Anspruch 1, wobei die Objektfunktionsbereitstellungseinheit (7) vorzugsweise dafür ausgelegt ist, eine Spline-Linienfunktion, die benachbarte x-z-Positionen (9, 20...25) verbindet, als die Objektfunktion (8) bereitzustellen.

4. Objektortungsgerät nach Anspruch 1, wobei die Objektfunktion (8) mindestens ein die Objektfunktion (8) bildendes Objektfunktionselement (17, 26...30) umfasst, wobei das Objekt (4) mindestens zwei Markierungen (5) umfasst, wobei ein Objektfunktionselement (17, 26...30) zwei benachbarte x-z-Positionen verbindet, wobei die Objektfunktionsanpassungseinheit (12) konfiguriert ist, um die Objektfunktion (8) derartig anzupassen, dass mindestens eine der folgenden Bedingungen erfüllt ist:

   - zwei Objektfunktionselemente haben den gleichen Wert an einer x-z-Position, die zwei benachbarte x-z-Positionen hat,
   - die erste Ableitung von zwei Objektfunktionselementen ist an einer x-z-Position, die zwei benachbarte x-z-Positionen hat, gleich,
   - die zweite Ableitung von zwei Objektfunktionselementen ist an einer x-z-Position, die zwei benachbarte x-z-Positionen hat, gleich,
   - die zweite Ableitung eines Objektfunktionselements ist an einer x-z-Position, die nur eine benachbarte x-z-Position hat, null.

5. Objektortungsgerät nach Anspruch 1, wobei das Objektortungsgerät (1) weiterhin eine Markierungspositionsbereitstellungseinheit (18) zum Bereitstellen einer Markierungsposition von mindestens einer der Markierungen (5) auf dem Objekt (4) umfasst, wobei die Objektfunktionsanpassungseinheit (12) konfiguriert ist, um abhängig von der bereitgestellten Markierungsposition und der Position der entsprechenden erkannten projizierten Markierung in der Projektionsebene eine x-z-Position zu bestimmen, die der bereitgestellten Markierungsposition entspricht, und um die Objektfunktion derartig anzupassen, dass die bestimmte x-z-Position (9) auf der angepassten Objektfunktion liegt.

6. Objektortungsgerät nach Anspruch 1, wobei das Objekt (4) eine maximal mögliche Krümmung hat und wobei die Objektfunktionsanpassungseinheit (12) konfiguriert ist, um die Objektfunktion (8) derartig anzupassen, dass ihre Krümmung nicht größer ist als die maximal mögliche Krümmung des Objekts (4).

7. Objektortungsgerät nach Anspruch 1, wobei das Objektortungsgerät (1) weiterhin eine Hüllkurvenbereitstellungseinheit (19) zum Bereitstellen einer mögliche Positionen der Objektfunktion definierenden Hüllkurve umfasst, wobei die Objektfunktionsanpassungseinheit (12) konfiguriert ist, um die Objektfunktion derartig anzupassen, dass sie innerhalb der Hüllkurve liegt.

8. Objektortungsgerät nach Anspruch 1, wobei die Positionsbestimmungseinheit (13) dafür ausgelegt ist, die Position des Objekts (4) zu bestimmen durch

- Bestimmen von zwei Dimensionen der Positionen der Markierungen (5) als die zweidimensionalen Positionen der erkannten projizierten Markierungen (10) in der Projektionsebene (6),
- Bestimmen einer dritten Dimension der Positionen der Markierungen (5) als die z-Positionen der x-z-Positionen, die den Markierungen (5) entsprechen, entlang der angepassten Objektfunktion (8),
- Anordnen der angepassten Objektfunktion (8) derartig, dass die x-z-Positionen entlang der angepassten Objektfunktion (8) mit den bestimmten dreidimensionalen Positionen der Markierungen (5) übereinstimmen.

9. Objektnavigationsgerät, das Folgendes umfasst:

- ein Objekt (4) mit Markierungen (5),
- ein Objektortungsgerät (1) nach Anspruch 1 zur Ortung des Objekts, wobei die Position des Objekts bestimmt wird,
- eine Objektnavigiereinheit (67) zum Navigieren des Objekts (4) abhängig von der bestimmten Position des Objekts.

10. Objektortungsverfahren zur Ortung eines Objekts mit Markierungen, wobei das Objektortungsverfahren Folgendes umfasst:

- Bereitstellen eines Projektionsbildes (3), das erzeugt wird, indem das Objekt (4) mit den Markierungen (5) in eine Projektionsebene (6) projiziert wird,
- Erkennen der projizierten Markierungen (10) und des projizierten Objekts (11) in dem Projektionsbild (3),
- Bereitstellen einer Objektfunktion (8) entlang x-z-Positionen (9, 20...25), wobei die Objektfunktion (8) das Objekt (4) darstellt und die x-z-Positionen (9, 20...25) durch Paare aus einer x-Position und einer z-Position definiert werden, wobei eine x-Position eine Position einer erkannten projizierten Markierung (10) entlang einer fiktiven Linie auf dem erkannten projizierten Objekt (11) in der Projektionsebene (6) ist und wobei eine z-Position eine Position in einer Richtung (40) definiert, die außerhalb der Projektionsebene (6) liegt,
- Modifizieren der Objektfunktion (8) derartig, dass Abstände zwischen den x-z-Positionen (9, 20...25) entlang der Objektfunktion (8) an Abstände zwischen den Markierungen (5) des Objekts (4) angepasst werden, indem die z-Positionen variiert werden,
- Bestimmen der Position des Objekts (4) basierend auf der angepassten Objektfunktion und den Positionen der erkannten projizierten Markierungen in der Projektionsebene.

11. Objektnavigationsverfahren, das Folgendes umfasst:

- Bereitstellen eines Objekts (4) mit Markierungen (5),
- Orten des Objekts (4) durch das Objektortungsverfahren nach Anspruch 10, wobei die Position des Objekts (4) bestimmt wird,
- Navigieren des Objekts (4) abhängig von der bestimmten Position des Objekts.

12. Objektortungscomputerprogramm zur Ortung eines Objekts mit Markierungen, wobei das Objektortungscomputerprogramm Programmcodemittel umfasst, die einen Computer zur Ausführung der Schritte des Objektortungsverfahrens nach Anspruch 10 veranlassen, wenn das Computerprogramm auf einem Computer ausgeführt wird, der ein Objektortungsgerät nach Anspruch 1 steuert.

13. Objektnavigationscomputerprogramm, wobei das Objektnavigationscomputerprogramm Programmcodemittel umfasst, die einen Computer zur Ausführung der Schritte des Objektnavigationsverfahrens nach Anspruch 11 veranlassen, wenn das Computerprogramm auf einem Computer ausgeführt wird, der ein Objektnavigationsgerät nach Anspruch 9 steuert.


**Revendications**

1. Appareil de localisation d'objet servant à localiser un objet doté de marqueurs, l'appareil (1) de localisation d'objet comprenant :

- une unité (2) fournissant une image de projection servant à fournir une image de projection (3) générée par la projection de l'objet (4) avec les marqueurs (5) dans un plan de projection (6),
- une unité (34) de reconnaissance d'image de projection servant à reconnaître les marqueurs projetés (10) et l'objet projeté (11) sur l'image de projection (3),
- une unité (7) fournissant une fonction d'objet servant à fournir une fonction d'objet (8) le long des positions x-z (9, 20...25), la fonction d'objet (8) représentant l'objet (4) et les positions x-z (9, 20...25) étant définies par des paires d'une position x et d'une position z, une position x étant une position d'un marqueur projeté (10) reconnu le long d'une ligne fictive (41) sur l'objet projeté (11) reconnu dans le plan de projection (6) et une position z définissant une position dans une direction (40) se trouvant hors du plan de projection (6),
- une unité (12) d'adaptation de fonction d'objet servant à modifier la fonction d'objet (8) de façon que les distances entre les positions x-z (9, 20...25) le long de la fonction d'objet (8) soient adaptées aux distances entre les marqueurs (5) de l'objet (4) par la variation des positions z,
- une unité (13) de détermination de position servant à déterminer la position de l'objet (4) sur la base de la fonction d'objet adaptée et des positions des marqueurs projetés reconnus dans le plan de projection.

2. Appareil de localisation d'objet selon la revendication 1, dans lequel l'unité (12) d'adaptation de fonction d'objet est conçue pour modifier la fonction d'objet (8) de façon que les distances entre les positions x-z (9, 20...25) le long de la fonction d'objet (8) soient adaptées aux distances entre les marqueurs (5) de l'objet (4) le long d'une ligne fictive (35) qui suit la forme de l'objet (4) et longe les marqueurs (5), par la variation des positions z.

3. Appareil de localisation d'objet selon la revendication 1, dans lequel l'unité (7) fournissant une fonction d'objet est conçue de préférence pour fournir, en tant que fonction d'objet (8), une fonction en ligne brisée connectant des positions x-z (9, 20...25) voisines.

4. Appareil de localisation d'objet selon la revendication 1, dans lequel la fonction d'objet (8) comprend au moins un élément de fonction d'objet (17, 26...30) formant la fonction d'objet (8), l'objet (4) comprenant au moins deux marqueurs (5), un élément de fonction d'objet (17, 26...30) connectant deux positions x-z voisines, l'unité (12) d'adaptation de fonction d'objet étant conçue pour adapter la fonction d'objet (8) de façon qu'au moins une des conditions suivantes soient remplies :

   - deux éléments de fonction d'objet possèdent la même valeur à une position x-z, qui a deux positions x-z voisines,
   - la dérivée première de deux éléments de fonction d'objet est égale à une position x-z, qui a deux positions x-z voisines,
   - la dérivée seconde de deux fonctions d'élément d'objet est égale à une position x-z, qui a deux positions x-z voisines,
   - la dérivée seconde d'un élément de fonction d'objet est nulle à une position x-z, qui n'a seulement qu'une position x-z voisine.

5. Appareil de localisation d'objet selon la revendication 1, dans lequel l'appareil (1) de localisation d'objet comprend en outre une unité (18) fournissant une position de marqueur servant à fournir une position de marqueur d'au moins un des marqueurs (5) sur l'objet (4), l'unité (12) d'adaptation de fonction d'objet étant conçue pour déterminer une position x-z qui correspond à la position de marqueur fournie en fonction de la position de marqueur fournie et de la position du marqueur projeté reconnu correspondant dans le plan de projection, et à adapter la fonction d'objet de façon que la position x-z (9) déterminée se trouve sur la fonction d'objet adaptée.

6. Appareil de localisation d'objet selon la revendication 1, dans lequel l'objet (4) présente une courbure maximale possible et dans lequel l'unité (12) d'adaptation de fonction d'objet est conçue pour adapter la fonction d'objet (8) de façon que sa courbure ne soit pas supérieure à la courbure maximale possible de l'objet (4).

7. Appareil de localisation d'objet selon la revendication 1, dans lequel l'appareil (1) de localisation d'objet comprend en outre une unité (19) fournissant une enveloppe servant à fournir une enveloppe définissant les positions possibles de la fonction d'objet, l'unité (12) d'adaptation de fonction d'objet étant conçue pour adapter la fonction d'objet de façon à ce qu'elle soit située au sein de l'enveloppe.

8. Appareil de localisation d'objet selon la revendication 1, dans lequel l'unité (13) de détermination de position est conçue pour déterminer la position de l'objet (4) par :

   - la détermination de deux dimensions des positions des marqueurs (5) en tant que positions bidimensionnelles des marqueurs projetés (10) reconnus dans le plan de projection (6),

- la détermination d'une troisième dimension des positions des marqueurs (5) en tant que positions z des positions x-z, qui correspondent aux marqueurs (5), le long de la fonction d'objet (8) adaptée,
- l'agencement de la fonction d'objet (8) adaptée de façon que les positions x-z le long de la fonction d'objet (8) adaptée coïncident avec les positions tridimensionnelles déterminées des marqueurs (5).

9. Appareil de guidage d'objet, comprenant :

- un objet (4) doté de marqueurs (5),
- un appareil (1) de localisation d'objet tel que défini dans la revendication 1 servant à localiser l'objet, dans lequel la position de l'objet est déterminée,
- une unité (67) de guidage d'objet servant à guider l'objet (4) en fonction de la position déterminée de l'objet.

10. Procédé de localisation d'objet servant à localiser un objet doté de marqueurs, le procédé de localisation d'objet comprenant :

- l'obtention d'une image de projection (3) générée par la projection de l'objet (4) avec les marqueurs (5) dans un plan de projection (6),
- la reconnaissance des marqueurs projetés (10) et de l'objet projeté (11) sur l'image de projection (3),
- l'obtention d'une fonction d'objet (8) le long de positions x-z (9, 20...25), la fonction d'objet (8) représentant l'objet (4) et les positions x-z (9, 20...25) étant définies par des paires d'une position x et d'une position z, une position x étant une position d'un marqueur projeté (10) reconnu le long d'une ligne fictive sur l'objet projeté (11) reconnu dans le plan de projection (6) et une position z définissant une position dans une direction (40) se trouvant hors du plan de projection (6),
- la modification de la fonction d'objet (8) de façon que les distances entre les positions x-z (9, 20...25) le long de la fonction d'objet (8) soient adaptées aux distances entre les marqueurs (5) de l'objet (4) par la variation des positions z,
- la détermination de la position de l'objet (4) sur la base de la fonction d'objet adaptée et des positions des marqueurs projetés reconnus dans le plan de projection.

11. Procédé de guidage d'objet, comprenant :

- l'obtention d'un objet (4) doté de marqueurs (5),
- la localisation de l'objet (4) par le procédé de localisation d'objet tel défini dans la revendication 10, dans lequel la position de l'objet (4) est déterminée,
- le guidage de l'objet (4) en fonction de la position déterminée de l'objet (4).

12. Programme informatique de localisation d'objet servant à localiser un objet doté de marqueurs, le programme informatique de localisation d'objet comprenant des moyens de code de programme servant à amener un ordinateur à mettre en oeuvre les étapes du procédé de localisation d'objet tel défini dans la revendication 10, quand le programme informatique est exécuté sur un ordinateur commandant un appareil de localisation d'objet tel que défini dans la revendication 1.

13. Programme informatique de guidage d'objet, le programme informatique de guidage d'objet comprenant des moyens de code de programme servant à amener un ordinateur à mettre en oeuvre les étapes du procédé de guidage d'objet tel que défini dans la revendication 11, quand le programme informatique est exécuté sur un ordinateur commandant un appareil de guidage d'objet tel que défini dans la revendication 9.

FIG. 1

# FIG. 2

# FIG. 3

# FIG. 4

FIG. 5

FIG. 6

101

102

103

104

105

FIG. 7

201

202

203

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- 3D Reconstruction of Coronary Stents in Vivo Based on Motion Compensated X-Ray Angiograms. **BABAK MOVASSAGHI.** MICCAI 2006. LNCS, 2006, vol. 4191, 177-184

- **T. NETSCH ; H. O. PEITGEN.** Scale-Space Signatures for the Detection of Clustered Microcalcifications in Digital Mammograms. *IEEE Trans. Med. Imag.,* 1999, vol. 18 (9), 774-786